# EUROPEAN PATENT SPECIFICATION

(11) **EP 2 367 515 B1**
(45) Date of publication and mention of the grant of the patent: **14.11.2012**
(21) Application number: 11703085.8
(22) Date of filing: 26.01.2011
(51) Int. Cl.: A61F 13/26

(54) **APPLICATOR FOR FEMININE HYGIENE DEVICES**
APPLIKATOR FÜR HYGIENEVORRICHTUNGEN FÜR DIE FRAU
APPLICATEUR POUR DISPOSITIFS D'HYGIENE FEMININE

(30) Priority: 29.01.2010 US 696774
(43) Date of publication of application: 28.09.2011
(73) Proprietor: The Procter & Gamble Company, Cincinnati, OH 45202 (US)
(72) Inventor: HASSE, Margaret, Henderson, Wyoming, OH45215 (US)
(74) Representative: Briatore, Andrea
(86) International application number: PCT/US2011/022480
(87) International publication number: WO 2011/094240

(56) References cited:
- EP-A1- 0 104 039
- US-A1- 2007 032 758

## Description

### FIELD OF THE INVENTION

The present invention relates to an improved applicator for feminine hygiene devices, and more particularly, to an improved applicator for feminine hygiene devices having an optimized design for improved cleanliness.

### BACKGROUND OF THE INVENTION

Feminine hygiene devices, such as tampons and pessaries, are generally used by women within the vagina for feminine needs, such as, *e.g.*, to absorb menstrual or other body exudates, for pelvic support, and/or for other feminine needs. Such feminine products can be inserted into the vagina digitally, such as, *e.g.*, by using a finger, or can be inserted into the vagina by using an applicator.

Applicators typically can comprise an insertion member and a plunger. The material to be expelled from the applicator, such as an absorbent tampon or pessary, can be positioned within the insertion member. The insertion member can have a first end for insertion of the material and a second end for receipt of the plunger. To use the applicator, the consumer can grasp the insertion member, position the first end appropriately, such as, *e.g*., into the body, and move the plunger in the insertion member towards the first end to insert the material. Some applicators can also include a fingergrip configuration that is located on the insertion member, which can allow the consumer to more securely hold the applicator during insertion of a material into the body cavity.

When the applicator is inserted into the body, residual fluid often remains in the body cavity. In the case of tampon use, residual fluid may result from fluid retained in the ruggae of the vaginal wall, from squeeze out of the soiled tampon upon removal, or from flow occurring in between the removal of the soiled tampon and the insertion of the clean one. This residual fluid soils the surface of the applicator when the next tampon is inserted.

Applicators are typically polyethylene or a wax coated paper. Residual fluid adheres to these applicator surfaces as a smear or coalesced droplets. This presents a significant hygiene problem to the user upon removal as the soiled applicator must be handled carefully to avoid spreading residual fluid to hands, garments and bathroom surfaces.

EP 104039 A1 describes a tampon applicator including a collapsible portion which is a water permeable micro apertures film.

Although many different types of applicators for feminine hygiene devices have been previously described, currently available configurations are not yet optimized to reduce the risk of contact with residual fluids upon removal from the body. As such, there remains a need for an improved applicator having an optimized design for improved cleanliness.

### SUMMARY OF THE INVENTION

The present invention solves the aforementioned need by providing an applicator for a feminine hygiene device, which has been optimized for improved cleanliness. While applicators of the prior art have used various techniques such as wrapping the applicator with a band of woven or non-woven material to absorb residual fluid, these attempts have largely failed to solve the problem of residual fluid transfer. Woven and non-woven materials trap residual fluids in interstitial spaces on the surface of the material and continue to allow transfer of small amounts of fluids via contact with the material as well as an unclean appearance from the visual presence of fluid. In addition, light application of force to the woven or non-woven material results in an expulsion of the absorbed residual fluid and provides the user with a distinctly negative experience.

The present invention solves the aforementioned problems via the use of a zone of micro-apertured film on the applicator which allows the residual fluid to pass through the micro-apertures of the film and become entrapped between the film and the applicator body. The residual fluid is removed from the surface and the fluid resists expulsion from light force back through the micro-apertures of the film. Applicant has surprisingly discovered that the use of the micro-apertured film of the present invention, which is itself effectively non-absorbing, in conjunction with an insertion member of an applicator, which is also effectively a non-absorbing body, can combine to effectively prevent residual fluids, such as urine and menses, from coming into contact with hands, garments or bathroom surfaces.

Accordingly, an applicator for a feminine hygiene device is provided. The applicator has an insertion member with an insertion end, a withdrawal end opposite the insertion end, and a barrel region adapted to house the feminine hygiene device. The barrel region is disposed between the insertion end and the withdrawal end and has an outer surface with a zone of micro-apertured polymeric film on it. The zone of micro-apertured film can cover from about 10% to about 100% of the area of the barrel region and from about 10% to about 100% of its circumference.

In addition, the micro-apertured film may have discrete protrusions which extend from the planar surface of the film with the protrusions having open distal ends and open proximal ends in the planar surface opposite the distal ends. The micro-apertured film may be oriented on the barrel region such that the open distal ends face the outer surface of the barrel region leaving the open proximal ends fluid facing. In additional embodiments of the present invention, a feminine hygiene article is provided wherein a feminine hygiene device is disposed in the barrel region of the applicator.

### BRIEF DESCRIPTION OF THE DRAWINGS

Fig. 1 is a side view of an embodiment of the present invention.
Fig. 2 is a cross sectional view of an embodiment of the present invention.
Fig. 3 is a greatly enlarged simplified perspective illustration of a segment of a micro-apertured film of the type generally described in the present invention.
Fig. 4 is a greatly enlarged simplified cross-sectional illustration of a segment of a micro-apertured film of the type generally described in the present invention.

### DETAILED DESCRIPTION OF THE INVENTION

Improved feminine hygiene device applicators are provided by the present invention, that can, for example, provide a consumer with improved cleanliness and/or improved perception of cleanliness during use of the applicator. In certain embodiments, the feminine hygiene device applicator can include an insertion member having a zone of a micro-apertured film on the insertion member. The zone of micro-apertured film on the applicator can be provided on a part or the entirety of a barrel region of the insertion member, and in preferred embodiments is a band that encircles the entire circumference of the barrel region. The zone of micro-apertured film may be provided on the outer surface of the applicator as a raised portion or may be provided so that the zone is flush with the applicator surface. Such applicator configurations can provide an improved cleanliness experience to the consumer and can also provide the perception of a more comfortable applicator.

As used herein, the term "feminine hygiene device" includes absorbent articles useful for feminine needs, such as articles that typically can be intended for feminine use internally, such as, e.g., within a user's vagina. Internal feminine hygiene devices can include, for example, tampons and pessaries.

As used herein, the term "tampon" refers to any type of absorbent structure that can be inserted into the vaginal canal or other body cavity, such as, *e.g.,* for the absorption of fluid, to aid in wound healing, and/or for the delivery of materials, such as moisture or active materials such as medicaments.

As used herein, the term "pessary" refers to any type of substantially non-absorbent structure for the purpose of reducing urine leakage and/or supporting a prolapsed uterus and/or bladder. Such pessaries can have any variety of shapes and sizes including cylinder, ovate, spherical, tubular, annual rings, "U" shaped, cup shaped, rings, cubes or donut shaped, and can function in any suitable manner, such as, *e.g*., by direct application of support, lever force, expansion of the device by selection of material, and/or by inflation of the device.

As used herein, the term "vaginal canal" refers to the internal genitalia of the human female in the pudendal region of the body. The terms "vaginal canal" or "within the vagina" as used herein are intended to refer to the space located between the introitus of the vagina (sometimes referred to as the sphincter of the vagina) and the cervix.

As used herein, "applicator" refers to a device or implement that facilitates the insertion of a feminine hygiene device, such as, *e.g*., a tampon or pessary, into an external orifice of a mammal. Exemplary applicators include telescoping, tube and plunger, and compact applicators.

As used herein, the term "insertion end" refers to the portion of the tampon or applicator including the end that is intended to enter the vaginal canal first when inserting the tampon or applicator into the vaginal canal.

As used herein, the term "withdrawal end" refers to the portion of the applicator opposite the insertion end including the end that is intended to exit the vaginal canal first when the applicator is removed from the vagina.

As used herein, the term "barrel region" refers to the portion of the applicator adapted to house the feminine hygiene device. In certain embodiments, the barrel region includes the region of the applicator having the largest diameter.

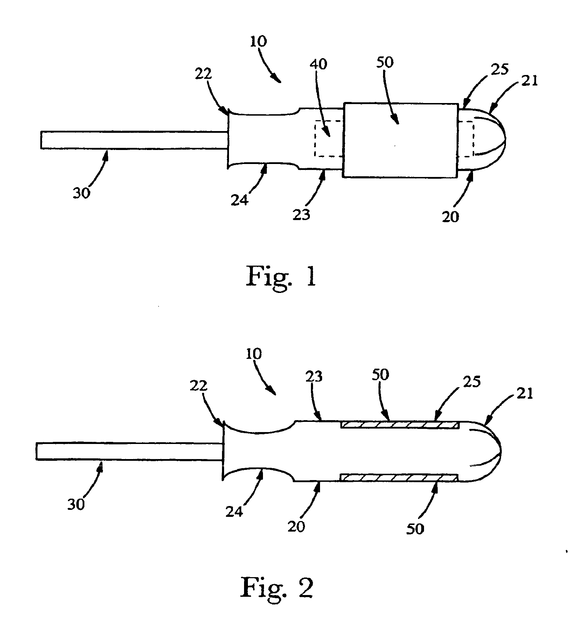

FIG. 1 shows one embodiment of an applicator 10. The applicator 10 comprises an insertion member 20 and a plunger 30. The insertion member 20 has an insertion end 21 and a withdrawal end 22 opposite the insertion end 21. The insertion member 20 can also include a barrel region 23 adapted to contain a feminine hygiene device such as, e.g., tampon 40. Feminine hygiene articles of the present invention comprise the device 40 placed with the applicator 10 and may optionally include a wrapper for enclosing the applicator and device or a package for sale to consumers. The barrel region 23 extends from the indention region 24 to the beginning of insertion end 21, which is generally defined as the beginning of formed weakness in the insertion end to allow the feminine hygiene device 40 to be pushed from the applicator 10. While, insertion member 20 and barrel region 23 are depicted as tubular, various other configurations are within the scope of the present invention as the desires of the consumer or optimized performance dictate. Numerous cross sectional shapes are within the scope of the present invention (e.g. square, triangular, rectangular, pentagonal, trapezoidal, arcuate, oval, circular, etc) and the specific geometry itself is not critical to the present invention. An indentation region 24 may also be provided opposite the insertion end 21, such as, e.g., proximal to the withdrawal end 22. As shown in Figure 1, the indentation region 24 protrudes inward from an outer surface 25 of the insertion member 20. As shown in Figure 1, indentation region 24 can, in certain embodiments, be disposed continuously about the circumference of the insertion member 20. A zone of micro-apertured film 50 is provided on the outer surface 25 of barrel region 23.

The zone of micro-apertured film 50 may be provided on the whole of barrel region 23 or on some smaller portion thereof as shown. Preferably from about 10% to about 100%, from about 25% to about 75%, from about 40% to about 60% of the area of barrel region 23 is covered and from about 10% to about 100%, about 25% to about 100% and about 50% to about 100% of its circumference. In preferred embodiments 50% of barrel region 23 is covered and 100% of its circumference. The zone 50 may encircle the barrel region 23 as a band as shown or be provided in a pattern or as a plurality of discrete regions as the desires of the consumer or optimized performance dictate. Suitable configurations include, but are not limited to, longitudinal strips, circumferential bans, waves, swirls or various patterns of individual regions. Accordingly, various shapes and patterns are envisioned within the scope of the present invention. Using standard film printing techniques available in the art, the film of zone 50 may be provided with printing to further enhance the aesthetic appearance of applicator 10. In preferred embodiments, a name, symbol, design or some indicia such as color or pattern is printed on the film.

The feminine hygiene device, the applicator, a wrapper surrounding the applicator, and/or an external package for sale can all be visually coordinated in some combination. For example, tampons having one or more visual signals or indicia such as a particular pattern, design, slogan, color or some combination can be disposed in applicators having a visual signal or indicia on the micro-apertured film 50 that corresponds in visual distinction to the color signals of the tampon and can be packaged in wrappers and/or boxes or cartons bearing a visual signal or indicia that corresponds in visual distinction to the color signals of the tampon. Thus, if the color signal is a shade of color, visual indicia on the wrapper and/or package can be a matching or substantially-matching shade of color. By "substantially-matching" is meant the color is close enough that the benefit indicator and the wrapper and/or packaging can be easily matched by one comparing tampons and packaging. For example, substantially-matching shades can be matching within the range of normal variance of colors from lot to lot of ink, dye, or other color-inducing medium, or within normal variance due to slight differences perceived on film versus paper, and the like. Other means of obtaining corresponding visual distinction include matching the shapes, styles, or overall appearance of visual indicia with corresponding benefit indicators. Thus, in certain embodiments, a user of feminine hygiene articles can choose a tampon having a desired feature more easily based on the wrapper and/or packaging, with a confirmation or reinforcement of that feature on each applicator inside the wrapper via coordination on the micro-apertured film 52. In certain embodiments, the articles can be co-packaged and/or co-marketed with one or more feminine hygiene articles, such as, e.g., a liner, a sanitary napkin, an interlabial pad, a wipe, or other suitable article. In addition, the article can be co-packaged and/or co-marketed with one or more feminine hygiene articles having one or more indicia that can coordinate and/or substantially match one or more benefit indicators on the article.

The zone of micro-apertured film 50 may be provided as a raised portion on barrel region 23 as shown in FIG. 1 or the barrel region 23 may be formed such that zone 50 is flush with barrel region 23 as shown in FIG. 2. The micro-apertured film 52 can be attached to barrel region 23 in any suitable manner, such as, for example, by adhesive bonding, such as, for example, using pressure sensitive adhesives, heat activated adhesives, hot melt adhesives, solvent based adhesives, water based adhesives, glue, or any other suitable adhesive, by mechanical bonding, by thermal bonding, by ultrasonic bonding, or in any other suitable manner. While a particular level of bonding need not be employed, the bonding should be sufficient such that the micro-apertured film 52 remains in place during insertion and removal of the applicator and that the edges and seams, if present, remain suitably bonded during use.

In preferred embodiments of the present invention the film 52 is adhered with a suitable adhesive as set forth above. The adhesive may be applied via various techniques such as for example continuous sheet, spiral or spray and may undertake various patterns or shapes. Even should continuous, spiral or spray application be employed the typical variation in height of the micro-apertured film allows the residual fluid to pass through the apertures in the film as set forth in detail herein. Accordingly, in particularly preferred embodiments of the present invention, a layered structure is provided of a plastic or paper based insertion member 20 having the micro-apertured film 52 adhered to the outer surface 25 of barrel region 23.

In additional embodiments, the indentation region 24 can comprise any suitable shape and/or configuration that can facilitate grasping and/or holding of the applicator. For example, the indention region can be a shape and/or configuration suitable for positioning one or more of a user's fingers within the indention region. For example, in certain embodiments, the indentation region can be disposed continuously about the circumference of the insertion member. The indentation region can have any suitable shape and/or cross-section, such as, e.g., circular, oval, elliptical, or a cross-section having a non-arcuate perimeter, such as, *e.g.,* a square, rectangular, triangular, polygonal, flattened, or other suitable cross-sectional shape. In certain embodiments, the indentation region can have a perimeter wherein a portion of the perimeter is arcuate and wherein a portion of the perimeter is non-arcuate, such as, *e.g*., an indentation region with one or more curved sides and one or more flattened sides.

The insertion member can be constructed from any suitable material. Suitable materials include, for example, paper, paperboard, cardboard, cellulose, such as, *e.g*., molded cellulose, or any combinations thereof; polymers such as polyethylene, polypropylene, polybutylene, polystyrene, polyvinylchloride, polyacrylate, polymethacrylate, polyacrylonitrile, polyacrylamide, polyamide, nylon, polyimide, polyester, polycarbonate, polylactic acid, poly hydroxyalkanoate, ethylene vinyl acetate, polyurethane, silicone, derivatives thereof, copolymers thereof, mixtures thereof, or any suitable smooth plastic material. Examples of suitable materials are disclosed in, *e.g*., U.S. Pat. Nos. 5,346,468 and 5,558,631. In certain embodiments, additives can be included in the material to alter or enhance certain material properties. Suitable additives include, for example, mold release agents, slip agents, surface energy modifiers, pearlescent agents, and/or any other suitable additives. In certain embodiments, the insertion member can be coated with a substance to give it a high slip characteristic, such as, *e.g*., with wax, polyethylene, a combination of wax and polyethylene, cellophane, clay, mica, and other lubricants that can facilitate comfortable insertion. Alternatively, or in addition, the insertion member can include a textured surface. Texture can be provided in any suitable manner, such as, *e.g*., by designing texture into or adding texture to the insertion member. In preferred embodiments the insertion member is formed to be liquid impervious and non-absorbent so as to protect the feminine hygiene device from moisture or other contaminants. Particularly, preferred are polymer materials for this purpose.

In certain embodiments, the insertion member 20 can be in the form of a spirally wound, convolutely wound or longitudinally seamed hollow tube, which can be formed from paper, paperboard, cardboard or a combination thereof. The insertion member can have one or more walls of any suitable thickness. In certain embodiments, the one or more walls can have a predetermined thickness of from about 0.1 millimeters to about 0.7 millimeter. The wall can be constructed from a single ply of material or can be formed from two or more plies that are bonded together, such as, *e.g*., to form a laminate. When two or more plies are utilized, some or all of the plies can be spirally wound, convolutely wound or longitudinally seamed to form an elongated cylinder. For example, in certain embodiments the wall can be constructed using a smooth thin ply of material on the outside or exterior surface that surrounds a coarser and possibly thicker ply. In embodiments where the wall contains at least three plies, the middle ply can be the thicker ply and the interior and exterior plies can be smooth and/or slippery to facilitate expulsion of the tampon and to facilitate insertion of the insertion member. The wall can contain one to four plies, although more plies can be utilized if desired.

The plies can be held together in any suitable manner, such as, *e.g*., by one or more adhesives, such as glue, by heat, by pressure, by ultrasonics, or by any other suitable manner for holding the plies together. The adhesive can be either water-soluble or water-insoluble. In certain embodiments, a water-soluble adhesive can be used such that the wall will quickly break apart when it is immersed in water, such as, *e.g*., by flushing the insertion member down a toilet.

Alternatively, the material can be overlapped into a tubular configuration, such as, for example, by spirally or convolutely winding the insertion member into a cylindrical tube. In the case of other tube construction methods such as fiber or plastic molding, or integral tube forming (*e.g*., thermoforming plastic) no seams may be present and the corrugations could optionally be formed as part of the tube molding or forming process.

As set forth herein, the insertion member 20 may also include an indentation region having a plurality of gripping formations, such as, *e.g.*, projections, rings, ridges, ribs, embossments, depressions, grooves, and/or other gripping structures. The gripping formations can be provided in any suitable manner, such as, *e.g.*, by the addition of material, and/or by impressing, such as, *e.g.*, by embossing, or compressing the surfaces. In certain embodiments, the indentation region can include one or more flattened sides and/or one or more spaces for a decorative marking or a character, such as, *e.g.*, an embossed and/or printed marking or character. In addition, or alternatively, the surfaces of the indentation region can include a material that can provide a frictional resistance for the user's fingers during the insertion of the tampon applicator into the body. Suitable materials that can provide friction include, for example, abrasive materials, high wet coefficient of friction materials, pressure sensitive adhesives, or any combinations thereof.

Typical dimensions for each of the insertion member and the plunger range from about 50 to about 100 mm and a diameter of from about 6 to about 22 mm and a thickness of from about 0.1 to about 0.7 mm.

The micro-apertured film 52 of the present invention allows for free transfer of fluids it comes into contact with through the apertures while inhibiting the reverse flow of these same fluids thereby providing a cleaner more sanitary applicator surface. While not wishing to be bound by theory, it is believed that fluids pass through the apertures of the film and become retained beneath the non-apertured surface of the film. Despite the lack of an absorption layer beneath the film in preferred embodiments, the fluid is retained by the zone 50 of micro-apertured film even with the application of the light pressures commonly exerted during the removal, handling and disposal of an applicator during use. The present invention in particularly preferred embodiments can transfer from about 0 to about 150 mm³, from about 25 mm³ to about 125 mm³ of residual fluid into which it comes into contact with. Thus, the size of zone 50 may be designed in conjunction with the film employed to tailor the transfer capacity with the desired range. By way of example, a zone 50 being 25.4 mm in width and encircling an applicator of diameter 14.5 mm employing a film 52 with protrusions of a height of 50 micron has a transfer capacity of about 57 mm³. Transfer capacity is measured as width multiplied by circumference multiplied by height of the protrusions.

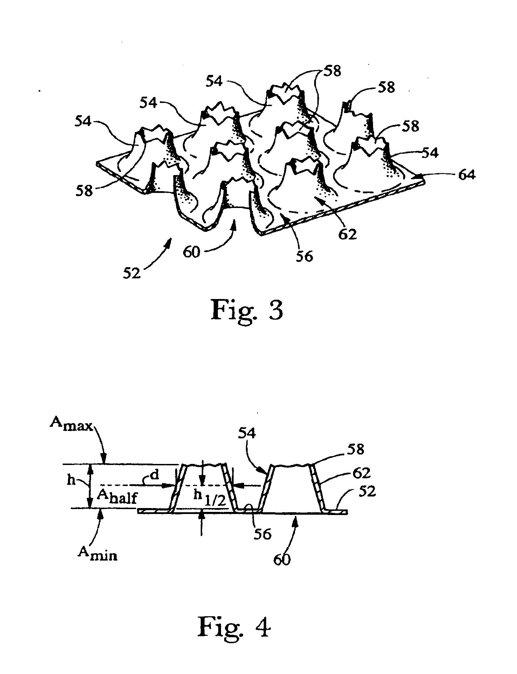

Turning to FIG. 3, the micro-apertured film 52 of the present invention is shown. The film 52 comprises a plurality of discrete protrusions 54 which extend from the planar surface 56 of the film. The discrete protrusions 54 have open distal ends 58 and open proximal portions 60 which in combination form the apertures in the film. The number, size, and distribution of discrete protrusions 54 on the film 52 can be predetermined based on various factors such as desired smoothness, soft feel, performance, etc. Turning to FIG. 4, the discrete protrusions 54 can be described as having a side wall(s) 62 defining the open proximal portion 60 and open distal end 58.

The discrete protrusions 54 each have a height h measured from minimum amplitude Aₘᵢₙ between adjacent protrusions to maximum amplitude Aₘₐₓ at the open distal end 58. The discrete protrusions have a diameter d, which for a generally cylindrical structure is the outside diameter at a lateral cross-section. By "lateral" is meant generally parallel to the plane of the first surface 56. For generally columnar discrete protrusions having non-uniform lateral cross-sections, and/or non-cylindrical structures of discrete protrusions, diameter d is measured as the average lateral cross-sectional dimension at ½ the height h of the discrete extended element, as shown in FIG. 4. Thus, for each discrete protrusion 54, an aspect ratio, defined as h/d, can be determined. The discrete protrusion 54 can have an aspect ratio h/d of at least about 3:1, at least about 2:1, at least about 1:1, at least about 0.75:1, or at least about 0.5:1. The discrete protrusions 54 will typically have an average height h of between about 25 microns and about 300 microns, between about 50 microns to about 200 microns, between 70 microns and about 150 microns. The discrete protrusions 54 will typically have a average diameter d of from about 50 microns to about 400 microns, from about 65 microns to about 200 microns, or from about 75 microns to about 150 microns. For protrusions that have generally non-columnar or irregular shapes, a diameter of the protrusion can be defined as two times the radius of gyration of the discrete protrusion at ½ height.

In general, because the actual height h of any discrete protrusion 54 can be difficult to determine, and because the actual height may vary, an average height h_{avg} of a plurality of discrete protrusions can be determined, and than be averaged. Such average height h_{avg} will typically fall within the ranges of heights described above. Likewise, for varying cross-sectional dimensions, an average diameter d_{avg} can be determined for a plurality of discrete protrusions 54. Such average diameter d_{avg} will typically fall within the ranges of diameters described above. Therefore, an average aspect ratio AR_{avg} of the discrete protrusions 54 for a predetermined portion of the film 52 can be expressed as h_{avg}/d_{avg}. The dimensions h and d for the discrete protrusions 54 can be indirectly determined based on the known dimensions of a forming structure, if the precursor web is fully conformed to the forming structure.

In a preferred embodiment, the diameter of a discrete protrusion 54 decreases with increasing amplitude (amplitude increases to a maximum at open distal end 58). As shown in FIG.'s 3 and 4, for example, the diameter, or average lateral cross-sectional dimension, of the discrete protrusion 54 can be a maximum at open proximal portion 60 and the lateral cross-sectional dimension steadily decreases to open distal end 58, which is smaller than the open proximal end. As a result a generally conical or volcano aperture is formed in the surface of the film. Alternatively, a mushroom arrangement may also be obtained. In certain embodiments of the present invention, it is preferred that the open distal end 58 is oriented such that it faces the outer surface 25 of the barrel region and the open proximal portion 60 of the aperture comes in contact with the fluid. Thus, the film 52 is oriented such that an inner surface 64 is adhered to outer surface 25 of the barrel region 23.

The "area density" of the apertures, which is the number of discrete apertures per unit area can be optimized and the film of the present invention will typically comprise from about 550 to about 2200, from about 750 to about 1900, from about 950 to about 1750, or from about 1100 to about 1600 discrete apertures per square centimeter. Such aperture counts per area can be made by any method known in the art, such as by optical microscopy.

The film of the present invention is produced from a precursor web of polymeric film, which can be a single layer of web material or a multilayer coextruded or laminate web material. Suitable polymeric films include thermoplastic films such as polyethylene, polypropylene, polystyrene, polyethylene terephthalate (PET), polymethylmethacrylate (PMMA), polyvinyl alcohol (PVA), nylon, polytetrafluoroethylene (PTFE) (e.g., TEFLON), or combinations thereof. Suitable polymeric films can comprise blends or mixtures of polymers. In certain embodiments, the precursor web can be a web comprising a sustainable polymer, such as polylactides, polyglycolides, polyhydroxyalkanoates, polysaccharides, polycaprolactones, and the like, or mixtures thereof.

The thickness of the precursor web prior to aperture formation will typically range from about 10 microns to about 150 microns, from about 15 microns to about 100 microns, or from about 20 microns to about 50 microns. Precursor polymeric webs will typically have a glass transition temperature of from about -100°C to about 120°C, from about -80°C to about 100°C, or other suitable ranges and optionally, may be plasticized to make it less brittle prior to processing.

The precursor web can be any polymeric film, having sufficient material properties to be formed into a micro-apertured film as described herein. The precursor web will typically have a yield point and the precursor web is preferably stretched beyond its yield point by the process of the present invention. That is, the precursor web should have sufficient yield properties such that the precursor web can be strained and rupture to form open distal ends. As disclosed below, process conditions such as temperature can be varied for a given polymer to permit it to stretch with rupture to form the film of the present invention. One material found suitable for use as a precursor web of the present invention is a 25 micron thick polyethylene film.

The precursor web can be a laminate of two or more webs, and can be a cho-extruded laminate. For example, precursor web can comprise two layers, and precursor web can comprise three layers, wherein the innermost layer is referred to as a core layer, and the two outermost layers are referred to as skin layers. In one embodiment, the precursor web comprises a three layer coextruded laminate having an overall thickness of about 25 microns, with the core layer having a thickness of about 18 microns; and each skin layer having a thickness of about 3.5 microns. The thickness of precursor web can be about 15 microns, 20 microns, 25 microns, 30 microns, 35 microns, 40 microns, 45 microns, or 60 microns. In one embodiment, the layers can comprise polymers having different stress-strain and/or elastic properties.

In certain embodiments, the precursor web can optionally further comprises a surfactant. If utilized, preferred surfactants include those from non-ionic families such as: alcohol ethoxylates, alkylphenol ethoxylates, carboxylic acid esters, glycerol esters, polyoxyethylene esters of fatty acids, polyoxyethylene esters of aliphatic carboxylic acids related to abietic acid, anhydrosorbitol esters, ethoxylated anhydrosorbitol esters, ethoxylated natural fats, oils, and waxes, glycol esters of fatty acids, carboxylic amides, diethanolamine condensates, and polyalkyleneoxide block copolymers. Molecular weights of surfactants selected for the present invention may range from about 200 grams per mole to about 10,000 grams per mole. Preferred surfactants have a molecular weight from about 300 to about 1,000 grams per mole.

If utilized, the surfactant level initially blended into the precursor web can be as much as 10 percent by weight of the total precursor web. Surfactants in the preferred molecular weight range (300-1,000 grams/mole) can be added at lower levels, generally at or below about 5 weight percent of the total precursor web.

In certain embodiments, the precursor web can also comprise titanium dioxide in the polymer blend. Titanium dioxide can provide for greater opacity of the embossed web. Titanium dioxide can be added at up to about 10 percent by weight of the precursor web, such as low-density polyethylene.

Other additives, such as particulate material, e.g., carbon black, iron oxide, mica, calcium carbonate (CaCO₃), particulate skin treatments or protectants, or odor-absorbing actives, e.g., zeolites, can optionally be added in one or more layers of precursor web. In some embodiments, embossed webs comprising particulate matter, when used in skin-contacting applications, can permit actives to contact the skin in a very direct and efficient manner. Specifically, in some embodiments, formation of discrete protrusions can expose particulate matter at or near the distal ends thereof. Therefore, actives such as skin care agents can be localized at or near either open distal ends or open proximal portions of the discrete protrusions to permit direct skin contact with such skin care agents when the film is used in skin contacting applications.

The average particle size of the particulate material, if utilized in the precursor web, will typically be from about 5 microns to about 100 microns. The use of certain particulate materials, such as mica particles, can dramatically improve the visual appearance of the embossed web.

The precursor web can also optionally comprise colorants such as dyes to improve the visual appearance of the embossed web. The precursor web can also optionally comprise fillers, plasticizers, and the like.

Various processes may form the apertured film of the present invention as known in the art including but not limited to, needle-punching, vacuum formation or hydroforming. The preferred film of the present invention as shown in FIG.3, has volcano-shaped micro-apertures, formed by a hydroforming process. A suitable hydroforming process is the first phase of the multiphase hydroforming process disclosed in U.S. Patent No. 4,609,518, issued to Curro et al. on September 2, 1986. An additional suitable manufacturing method disclosing the use of intermeshing rolls to produce apertured webs is set forth in U.S. Patent Publication 2006/0087053 .

### TEST METHOD

Images for dimensional measurements are acquired using a Scanning Electron Microscope (SEM) such as a Hitachi S3500N (Hitachi, Pleasanton, CA). Specific dimensions are manually measured on the acquired images using an image analysis software program such as Quartz PCI version 4.2 (Quartz Imaging Corporation, Vancouver, BC). The software is calibrated against a standard grid of known dimensions that has been previously analyzed by the SEM.

To prepare the specimen, the micro-apertured film sample is carefully removed from the applicator, such that the film and protrusions are not significantly deformed. If necessary, chlorofluorocarbon freeze spray (e.g. Cyto-Freeze, Control Company, Houston TX) can be used to facilitate removal. A piece of the film approximately 10 mm by 10 mm is cut from the sample and the edges are fractured with a razor blade while held under liquid nitrogen. The fracture line should be placed parallel to a row of un-sectioned cones that are to be measured. The fractured film is then adhered to a SEM mount with the surface that was proximal to the applicator affixed to the mount. The SEM mount with specimen is placed into a vacuum deposition system (such as a Desk II, Denton Vacuum, Moorestown, NJ) and Au sputter coated. The specimen is mounted into the SEM which is operated in normal vacuum mode. After applying the vacuum, the specimen is tilted 90° (by tilting the SEM stage) to image the side profile of the protrusions. The image magnification should be adjusted to view 3 to 5 discrete protrusions along the edge of the specimen.

From an image of the side profile, and referring to FIG 3, the height h of a discrete protrusion 54 can be measured by drawing a reference line Aₘₐₓ at the maximum amplitude of the open distal end 58, and drawing a second reference line Aₘᵢₙ at the distal surface of the minimum amplitude between adjacent protrusions 56. Both reference lines should be drawn generally parallel to the plane of the surface 56. The vertical distance between these two reference lines is h and is reported to the nearest 1 micron. A third reference line A_{hatf} is drawn by calculating the h_{1/2} of the protrusion (i.e., the height h divided by 2) and drawing it across the protrusion generally parallel to the plane of the surface 56. The diameter d is measured as the cross section, outer surface to outer surface, of the protrusion 54 at A_{half}. These measurements are repeated for at least 3 discrete protrusions on this specimen. A separate specimen is prepared in like fashion to measure 3 more protrusions. The average height h_{avg} and diameter d_{avg} are calculated as the average of the six measurements and reported to the nearest 1 micron.

### EXAMPLE I

The preferred micro-apertured film of the present invention was made generally from a hydroforming process following the first phase of the multi-phase hydroforming process set forth in U.S. Patents 4,609,518 and 4,629,643 . The precursor web was a polyethylene film about 25 microns thick provided by Tredegar Film Products, Terre Haute, IN. The web was fed onto a forming structure at a speed of about 152 meters per minute and subjected to a high-pressure water jet. The water temperature was about 74 °C, the water pressure about 69 bar and the water flow about 15 liters per minute per cross machine direction cm of web width. The forming structure was a woven wire mesh screen having 94 µm diameter wires with openings of about 117.5 µm. The high-pressure jet causes the smooth flat web to assume the general contour of the pattern of the woven wire support member. In addition, because the interstices formed by the intersecting filaments are unsupported, the fluid jet causes rupture at those portions of the web that coincides with the interstices in the woven wire support structure thereby producing a "planar" micro-apertured film as set forth in FIG. 3 herein. This produced a film having a multiplicity of apertures, approximately 100 micron in diameter, at a density of about 40 apertures per linear cm in both directions and having a height of about 140 microns. The micro-apertured film was than wound up on a take up roll to be applied to the applicator.

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. An applicator for a feminine hygiene device comprising:
an insertion member;
the insertion member having an insertion end, a withdrawal end opposite the insertion end, and a barrel region adapted to house a feminine hygiene device;
the barrel region being disposed between the insertion end and the withdrawal end and having an outer surface; and
a zone of micro-apertured polymeric film on the outer surface of the barrel region **characterized in that** the micro-apertured polymeric film comprises discrete protrusions which extend from the planar surface of the film, the protrusions having open distal ends and open proximal portions in the planar surface opposite the open distal ends.

2. The applicator of claim 1 wherein the zone of micro-apertured polymeric film covers from about 40% to about 60% of the area of the barrel region and from about 50% to about 100% of the circumference of the barrel region.

3. The applicator of any of claims 1-2 wherein the micro-apertured polymeric film is oriented on the barrel region such that the open distal ends face the outer surface of the barrel region.

4. The applicator of any of claims 1-3 wherein a visual signal is provided on the micro-apertured polymeric film.

5. The applicator of any of claims 1-4 wherein the micro-apertured polymeric film comprises discrete protrusions which extend from the planar surface of said film, the discrete protrusions having average height of from about 5 microns to about 300 microns.

6. The applicator of any of claims 1-5 wherein the discrete protrusions have average diameter of from about 50 to about 200 microns.

7. The applicator of any of claims 1-6 wherein the micro-apertured polymeric film is bounded to the barrel region via adhesive bonding.

8. A feminine hygiene article comprising:
A) An applicator, according to claims 1-7 and,
B) a feminine hygiene device disposed in the barrel region of the applicator.

## Patentansprüche

1. Applikator für eine Vorrichtung für die weibliche Intimhygiene, umfassend:
ein Element zum Einführen;
wobei das Element zum Einführen ein Ende zum Einführen, ein Ende zum Herausnehmen, das dem Ende zum Einführen gegenüber liegt, und eine Hülsenregion aufweist, die dafür ausgelegt ist, eine Vorrichtung für die weibliche Intimhygiene aufzunehmen;
wobei die Hülsenregion zwischen dem Ende zum Einführen und dem Ende zum Herausnehmen angeordnet ist und eine Außenfläche aufweist; und
eine Zone einer mit Mikroöffnungen versehenen Polymerfolie auf der Außenfläche der Hülsenregion, **dadurch gekennzeichnet, dass** die mit Mikroöffnungen versehene Polymerfolie voneinander abgegrenzte Vorsprünge umfasst, die von der planen Oberfläche der Folie vorstehen, wobei die Vorsprünge offene distale Enden und offene proximale Abschnitte in der planen Oberfläche, die den offenen distalen Enden gegenüber liegen, aufweist.

2. Applikator nach Anspruch 1, wobei die Zone der mit Mikroöffnungen versehenen Polymerfolie etwa 40 % bis etwa 60 % der Fläche der Hülsenregion und etwa 50 % bis etwa 100 % des Umfangs der Hülsenregion abdeckt.

3. Applikator nach einem der Ansprüche 1 bis 2, wobei die mit Mikroöffnungen versehene Polymerfolie so auf der Hülsenregion ausgerichtet ist, dass die offenen distalen Enden auf die Außenfläche der Hülsenregion gerichtet sind.

4. Applikator nach einem der Ansprüche 1 bis 3, wobei ein optisches Signal an der mit Mikroöffnungen versehenen Folie vorgesehen ist.

5. Applikator nach einem der Ansprüche 1 bis 4, wobei die mit Mikroöffnungen versehene Folie voneinander abgegrenzte Vorsprünge umfasst, die von der planen Oberfläche der Folie ausgehen, wobei die voneinander abgegrenzten Vorsprünge eine durchschnittliche Höhe von etwa 5 Mikrometer bis etwa 300 Mikrometer aufweisen.

6. Applikator nach einem der Ansprüche 1 bis 5, wobei die voneinander abgegrenzten Vorsprünge einen durchschnittlichen Durchmesser von etwa 50 bis etwa 200 Mikrometer aufweisen.

7. Applikator nach einem der Ansprüche 1 bis 6, wobei die mit Mikroöffnungen versehene Folie durch Verkleben an die Hülsenregion gebunden ist.

8. Artikel für die weibliche Intimhygiene, umfassend:
A) einen Applikator nach einem der Ansprüche 1 bis 7; und
B) eine Vorrichtung für die weibliche Intimhygiene, die in der Hülsenregion des Applikators angeordnet ist.

## Revendications

1. Applicateur pour un dispositif hygiénique féminin comprenant :
un membre d'insertion ;
le membre d'insertion ayant une extrémité d'insertion, une extrémité de retrait opposée à l'extrémité d'insertion, et une région de cylindre adaptée pour loger un dispositif hygiénique féminin ;
la région de cylindre étant disposée entre l'extrémité d'insertion et l'extrémité de retrait et ayant une surface externe ; et
une zone de film polymère microperforé sur la surface externe de la région de cylindre, **caractérisé en ce que** le film polymère microperforé comprend des parties saillantes discrètes qui s'étendent à partir de la surface plane du film, les parties saillantes ayant des extrémités distales ouvertes et des parties proximales ouvertes dans la surface plane opposée aux extrémités distales ouvertes.

2. Applicateur selon la revendication 1, dans lequel la zone de film polymère microperforé couvre de 40 % à 60 % de l'aire de la région de cylindre et d'environ 50 % à environ 100 % de la circonférence de la région de cylindre.

3. Applicateur selon l'une quelconque des revendications 1 à 2, dans lequel le film polymère microperforé est orienté sur la région de cylindre de telle sorte que les extrémités distales ouvertes font face à la surface externe de la région de cylindre.

4. Applicateur selon l'une quelconque des revendications 1 à 3, dans lequel un signal visuel est fourni sur le film polymère microperforé.

5. Applicateur selon l'une quelconque des revendications 1 à 4, dans lequel ledit film polymère microperforé comprend des parties saillantes discrètes qui s'étendent à partir de la surface plane dudit film, les parties saillantes discrètes ayant une hauteur moyenne allant d'environ 5 microns à environ 300 microns.

6. Applicateur selon l'une quelconque des revendications 1 à 5, dans lequel les parties saillantes discrètes ont un diamètre moyen allant d'environ 50 à environ 200 microns.

7. Applicateur selon l'une quelconque des revendications 1 à 6, dans lequel le film polymère microperforé est lié à la région de cylindre par l'intermédiaire d'une liaison adhésive.

8. Article d'hygiène féminine comprenant :
A) un applicateur, selon les revendications 1 à 7 : et
B) un dispositif hygiénique féminin disposé dans la région de cylindre de l'applicateur.
